# EUROPEAN PATENT SPECIFICATION

(11) **EP 4 241 741 B1**
(45) Date of publication and mention of the grant of the patent: **28.10.2026**
(21) Application number: 23189083.1
(22) Date of filing: 14.08.2015
(51) Int. Cl.: A61F 2/966

(54) **ENDOPROSTHESIS DELIVERY SYSTEMS WITH IMPROVED RETRACTION**
ENDOPROTHESENEINFÜHRUNGSSYSTEME MIT VERBESSERTEM RÜCKZUG
SYSTÈMES DE PLACEMENT D'ENDOPROTHÈSE AVEC RÉTRACTION AMÉLIORÉE

(30) Priority: 15.08.2014 US 201462038000 P; 13.08.2015 US 201514826040
(43) Date of publication of application: 13.09.2023
(62) Document number(s) of the earlier application(s) in accordance with Art. 76 EPC: 21179648.7 / 3 909 550; 15756741.3 / 3 179 965
(73) Proprietor: W. L. Gore & Associates, Inc., Newark, DE 19711 (US)
(72) Inventor: KILGROW, Bret J., Newark, DE 19711 (US); KOVACH, Larry J., Newark, DE 19711 (US); SHORT, Brandon C., Newark, DE 19711 (US); TIMBIE, Anna F., Newark, DE 19711 (US)
(74) Representative: HGF

(56) References cited:
- WO-A1-2007/059293
- DE-B3- 102006 053 748
- US-B1- 6 264 689

## Description

### FIELD

The present disclosure generally relates to endoprostheses for treating diseases of the vasculature and similar anatomies, and more particularly, to endoprosthesis delivery systems with at least one end cap proximal to a distal tip of the endoprosthesis delivery system.

### BACKGROUND

Many endoprosthesis, such as, for example, stent-grafts, are constructed to reinforce, replace, bridge, or otherwise treat a part of a blood vessel. An endoprosthesis may guide blood flow through a lumen defined by a generally tubular interior of such a vessel. Other tubular endoprostheses are designed for use in other body regions, for example, the esophagus, ureters, gastrointestinal tract and various ducts. In many cases, endoprostheses are constrained within a covering member or sheath and delivered to the body region requiring treatment on the end of an elongate member. When the covering member is removed, as during deployment, the devices are expanded under force or self-expand to assume a larger diameter. After delivery and deployment of the endoprosthesis, the elongate member used to delivery and deploy the endoprosthesis is retracted into a tubular outer shaft and removed from the body. In some instances, it may be difficult to properly align the elongate member and endoprosthesis with the outer shaft, causing difficulties in retraction. Thus, improved endoprosthesis delivery systems are desirable.

US6264689 describes an implantable medical stent having a low profile for delivery through smaller lumens. The stent is constrained during delivery where the helically wrapped points of a diamond cell structure also include at least one crossed joint of intersecting strands to provide a reduced diameter. WO2007/059293 describes an intraluminal prosthesis having an inner sheath and a nose cone connected to the distal end thereof. A stopper is connected to the inner sheath and is spaced apart from the nose cone. An intraluminal prosthesis sheath surrounds and is moveable in relation to the inner sheath. An annular intraluminal prosthesis space is defined radially between the intraluminal prosthesis sheath and the inner sheath, between the stopper and the nose cone and contains a compressed intraluminal prosthesis. DE102006053748 describes an insert system for inserting and releasing an endovascular stent, having an internal tube guided inside an external tube that is moveable in relation thereto. A fixing system fixes a proximal end of a stent, and has a cover unit and a retaining unit located proximal to the cover unit.

### SUMMARY

The invention relates to combined tip and end cap as set out in claim 1, and to an endoprosthesis delivery system including the combined tip and end cap, as set out in claim 10. Selected further features are recited in the dependent claims. Endoprosthesis delivery systems in accordance with the present disclosure can comprise an elongate member, an endoprosthesis comprising a distal end, an end cap having a cylindrical body, wherein the end cap is coupled to the elongate member adjacent to the distal end of the endoprosthesis, and a covering member surrounding a portion of the endoprosthesis, wherein a distal end of the covering member extends beyond the distal end of the endoprosthesis, and wherein a proximal end of the end cap comprises a tapered profile.

Endoprosthesis delivery systems in accordance with the present disclosure further comprise a tip adjacent a distal end of the end cap. The tip can have a diameter larger than a diameter of the end cap. The tip and end cap are integrated. The end cap can comprise a region of reduced diameter located between the proximal end and the distal end of the end cap. The distal end of the covering member can be positioned in the region of reduced diameter. The proximal end of the tip can be spaced apart from the distal end of the end cap. The distal end of the covering member can be positioned between the proximal end of the tip and the distal end of the end cap. The proximal end of the end cap can comprise a fin. The fin can be angled relative to a longitudinal axis of the end cap, and the fin can fold upon retraction of the end cap into the catheter The covering member can comprise a plurality of knit fibers, which can be woven about an exterior surface of the endoprosthesis and constrain the endoprosthesis in a constrained configuration. The delivery system can further comprise a pull line coupled to the covering member. The endoprosthesis can be a stent or stent-graft or similar device. The elongate member can be a guide wire. The delivery system can further comprise a catheter.

### BRIEF DESCRIPTION OF THE DRAWINGS

The features and advantages of the present disclosure will become more apparent from the detailed description set forth below when taken in conjunction with the drawings, wherein:
Figure 1 illustrates a perspective view of an endoprosthesis delivery system in accordance with the claims;
Figure 2 illustrates a perspective view of a combined tip and end cap of an endoprosthesis delivery system in accordance with the claims;
Figures 3A, 3B, and 3C illustrate partial cross sectional views of combined tips and end caps of endoprosthesis delivery systems;
Figures 4A, 4B, and 4C illustrate side views of combined tips and end caps of endoprosthesis delivery systems. The embodiments of Figures 4B and 4C are in accordance with the claims;
Figure 5 illustrates a magnified perspective view of a portion of an end cap of an endoprosthesis delivery system in accordance with the claims; and
Figure 6 illustrates a magnified perspective view of a portion of an end cap of another endoprosthesis delivery system in accordance with the claims.

### DETAILED DESCRIPTION

Persons skilled in the art will readily appreciate that various aspects of the present disclosure can be realized by any number of methods and apparatuses configured to perform the intended functions. Stated differently, other methods and apparatuses can be incorporated herein to perform the intended functions. It should also be noted that the accompanying drawing figures referred to herein are not all drawn to scale, but may be exaggerated to illustrate various aspects of the present disclosure, and in that regard, the drawing figures should not be construed as limiting.

The terms "endoprosthetic device," "endoprosthesis," "vascular device," and the like can refer, throughout the specification and in the claims, to any medical device capable of being implanted and/or deployed within a body lumen. In various instances, an endoprosthesis can comprise a stent, a stent-graft, graft, a filter, an occluder, a balloon, a lead, and energy transmission device, a deployable patch, an indwelling catheter, and the like.

In addition, throughout this specification and claims, the delivery systems described herein can, in general, include an endoprosthesis constrained by a "covering member" or "sheath." The covering member or sheath can, in various embodiments, comprise a sheet of material that is fitted about an endoprosthesis. The covering member or sheath can, in various embodiments, comprise a plurality of knitted fibers located about the endoprosthesis. These fibers can, for example, comprise a woven warp knit or knit-braid, as described in United States Patent No. 6,315,792 to Armstrong et al., issued November 13, 2001, entitled "Remotely removable covering and support,". The covering member can be coupled to a pull line extending down the length of the catheter, which a clinician can pull to facilitate uncovering the endoprosthesis.

For example, a covering member comprising a plurality of fibers can be coupled to a pull line, which a clinician can pull to unravel the plurality of fibers. Thus, the covering member can be characterized as "unzipped", in that the pull line causes the covering member to open or unzip along a straight line. In addition, in various embodiments, a covering member can be unzipped, first, along a proximal vector and, second, along a distal vector. In various embodiments, a covering member can be unzipped along a longitudinal vector running substantially parallel to the longitudinal axis of an elongate member.

As used throughout the specification and in the claims, the term "elongate member" can refer to a shaft-like structure such as a catheter, guidewire, or the like. In various embodiments, an endoprosthesis can be mounted or loaded on a catheter, also referred to herein as an inner shaft.

As used throughout the specification and in the claims, the term "outer shaft" can refer to a tubular element comprising a lumen, into which the endoprosthesis in a constrained diameter may be inserted and delivered into the body of a patient. Outer shafts can comprise, for example, an introducer sheath, among other suitable constructs.

Further, the term "distal" refers to a relative location that is farther from a location in the body at which the medical device was introduced. Similarly, the term "distally" refers to a direction away from a location in the body at which the medical device was introduced.

The term "proximal" refers to a relative location that is closer to the location in the body at which the medical device was introduced. Similarly, the term "proximally" refers to a direction towards a location in the body at which the medical device was introduced.

With continuing regard to the terms proximal and distal, this disclosure should not be narrowly construed with respect to these terms. Rather, the devices and methods described herein may be altered and/or adjusted relative to the anatomy of a patient.

As used herein, the term "constrain" may mean (i) to limit expansion, occurring either through self-expansion or expansion assisted by a device, of the diameter of an expandable implant, or (ii) to cover or surround an expandable implant (e.g., for storage or biocompatibility reasons and/or to provide protection to the expandable implant and/or the vasculature).

As used herein, the term "integral" refers to elements or components which are connected and/or coupled to each other such that they form a single physical object or structure.

In various embodiments, an endoprosthesis delivery system can comprise an elongate member, such as a catheter, an endoprosthesis, a covering member disposed about the endoprosthesis, a tip, and an end cap with a distally-tapered profile. The covering member can extend beyond an end of the endoprosthesis and on to the tip and/or end cap. The tip and/or end cap can comprise a region of reduced diameter. Further, the distal end of the covering member can terminate within the region of reduced diameter.

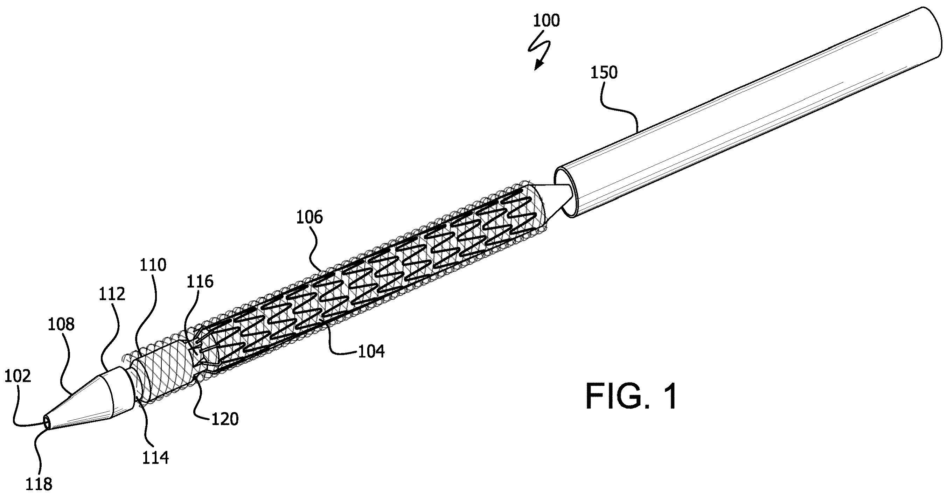

With initial reference to Figure 1, an endoprosthesis delivery system 100 can comprise, in various instances, an elongate member 102 onto which an endoprosthesis 104 is mounted. Elongate member 102 and endoprosthesis 104 can be surrounded by an outer shaft 150, such as an introducer sheath, or other tubular shaft member. In various instances, elongate member 102 and endoprosthesis 104 are passed through outer shaft 150 to deliver endoprosthesis 104 to a desired treatment area of a patient.

In various instances, endoprosthesis 104 comprises a compressible medical device. For example, in various embodiments, endoprosthesis 104 comprises a stent or stent-graft. Conventional stent-grafts are designed to dilate from their delivery diameter, through a range of intermediary diameters, up to a maximal, pre-determined functional diameter, and generally comprise one or more stent components with one or more graft members covering all or part of the inner and/or outer surfaces of the stent.

In various embodiments, endoprosthesis 104 comprises one or more stent components made of nitinol and a graft member made of ePTFE. However, and as discussed below, any suitable combination of stent component(s) and graft member(s) is within the scope of the present disclosure.

For example, stent components can have various configurations such as, for example, rings, cut tubes, wound wires (or ribbons) or flat patterned sheets rolled into a tubular form. Stent components can be formed from metallic, polymeric or natural materials and can comprise conventional medical grade materials such as nylon, polyacrylamide, polycarbonate, polyethylene, polyformaldehyde, polymethylmethacrylate, polypropylene, polytetrafluoroethylene, polytrifluorochlorethylene, polyvinylchloride, polyurethane, elastomeric organosilicon polymers; metals such as stainless steels, cobalt-chromium alloys and nitinol and biologically derived materials such as bovine arteries/veins, pericardium and collagen. Stent components can also comprise bioresorbable materials such as poly(amino acids), poly(anhydrides), poly(caprolactones), poly(lactic/glycolic acid) polymers, poly(hydroxybutyrates) and poly(orthoesters). Any expandable stent component configuration which can be delivered by a catheter is in accordance with the present disclosure.

Potential materials for graft members include, for example, expanded polytetrafluoroethylene (ePTFE), polyester, polyurethane, fluoropolymers, such as perfluoroelastomers and the like, polytetrafluoroethylene, silicones, urethanes, ultra high molecular weight polyethylene, aramid fibers, and combinations thereof. Other embodiments for a graft member material can include high strength polymer fibers such as ultra high molecular weight polyethylene fibers or aramid fibers. Further, a graft may comprise a class of polyesters such as polyethylene terephthalate and polyaramids, polyfluorocarbons such as polytetrafluoroethylene (PTFE) with and without copolymerized hexafluoropropylene, and porous or nonporous polyurethanes. Any graft member that can be delivered by a catheter is in accordance with the present disclosure.

System 100 further comprises a covering member 106 surrounding at least a portion of endoprosthesis 104 and maintaining endoprosthesis 104 in a constrained configuration. For example, covering member 106 can comprise a plurality of woven warp knit or knit-braid fibers. In such embodiments, the distal and/or proximal end regions of covering member 106 can be longitudinally compressed against the end(s) of endoprosthesis 104.

System 100 comprises a catheter tip 108 positioned at a distal end of elongate member 102 and distal to a distal end of endoprosthesis 104. Catheter tip 108 comprises a distal end 118 having a tapered profile. Catheter tip 108 further comprises a proximal end 120.

System 100 further comprises an end cap 110. End cap 110 may prevent covering member 106 from hanging-up on the endoprosthesis during deployment of endoprosthesis 104 (i.e., removal of the covering member from around endoprosthesis 104). End cap 110 can comprise, for example, a mildly or moderately deformable material, i.e., a low durometer polymeric material at least on the section closest to the endoprosthesis. For example, the low durometer polymeric material can have a durometer between 15 and 70 Shore on the Type A scale. In various embodiments, the end cap 110 can be constructed so as to be more compliant along the axis parallel to the longitudinal axis of the guiding member than across its radial dimension.

End cap 110 comprises a cylindrical body 112. End cap 110 can be located distal and adjacent to a distal end of endoprosthesis 104. In various embodiments, endoprosthesis 104 can have a distal edge which can abut or be disposed adjacent to end cap 110. In embodiments, end cap 110 is integral to catheter tip 108. In other example not according to the claims, end cap 110 and catheter tip 108 are distinct elements and are not integral to each other. Further, end cap 110 and catheter tip 108 can in other examples not according to the claims be spaced apart from one another, such that there is a gap or space between proximal end 120 of catheter tip 108 and a distal end of end cap 110.

End cap 110 can further comprise a region of reduced diameter 114. In various embodiments, region of reduced diameter 114 is located between distal end 118 and proximal end 120 of end cap 110. For example, region of reduced diameter 114 can comprise a cylindrical portion of end cap 110 that has a diameter less than that of cylindrical body 112.

Covering member 106 extends beyond the distal end of endoprosthesis 104 and onto end cap 110. For example, the distal end of covering member 106 can be positioned along a region of reduced diameter 114. The covering member 106 may encase a region of reduced diameter 114 tightly and therefore have an effective constrained diameter towards the distal end 118 of end cap 110 that is less than an effective constrained diameter of the covering member 106 around a ridge 122. In various embodiments, a portion of the distal end of covering member 106 can comprise a diameter less than that of cylindrical body 112 and equal to or greater than that of region of reduced diameter 114. In such embodiments, the distal end of covering member 106 can be secured within region of reduced diameter 114 or perhaps along ridges 122, which may assist in maintaining the position of the distal end during delivery of endoprosthesis 104 and/or prevent premature or undesired retraction of the distal end of covering member 106.

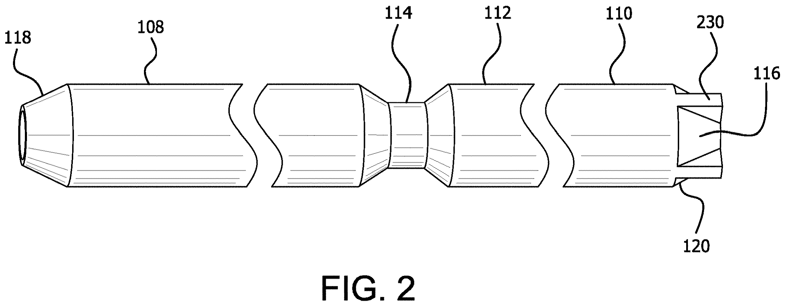

With reference to Figures 1-3B, proximal end 120 of end cap 110 can further comprise a profile 116. In various embodiments, after deployment of endoprosthesis 104, catheter tip 108 and end cap 110 are retracted back into outer shaft 150. Profile 116 may assist in retracting catheter tip 108 and end cap 110. For example, profile 116 can comprise a taper which may facilitate retraction of catheter tip 108 back into outer shaft 150 by providing an angled face of proximal end 120 instead of a flat and/or square face of proximal end 120.

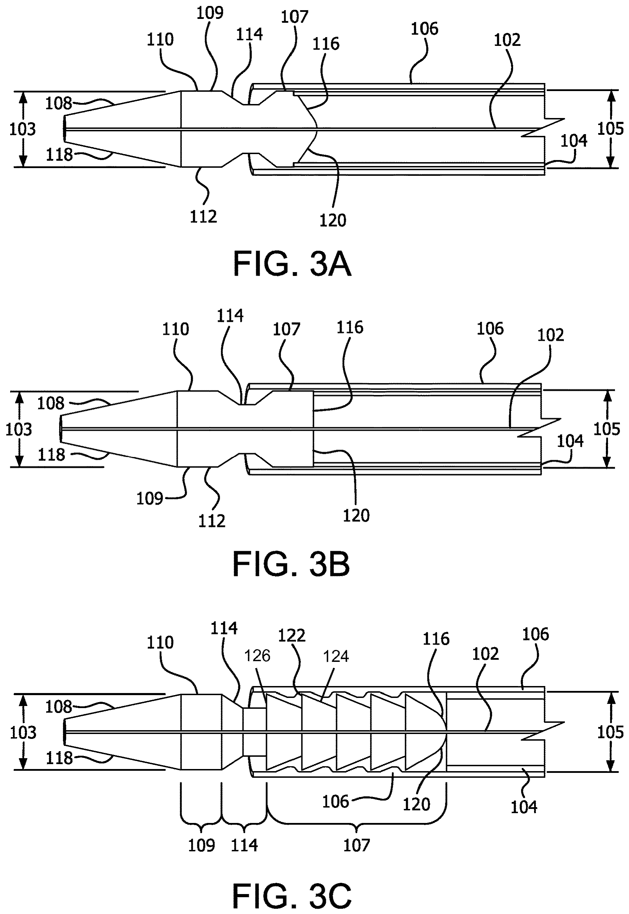

In certain instances, the end cap 110 may be described as including have various regions. For example, as shown in Figures 3A and 3B, the end cap 110 may have an end cap proximal region 107 and an end cap distal region 109. In some cases, as shown in Figures 3A and 3B, the end cap 110 may include an end cap reduced diameter region 114 between the end cap proximal region 107 and the end cap distal region 109. In addition, the end cap proximal region 107 may also include an end cap proximal region effective maximum diameter 105 and the end cap distal region 109 may include an end cap distal region effective maximum diameter 103.

In certain instances, the end cap 110 may comprise various features. For example, as shown in Figure 3C, the end cap 110 may comprise one or more (e.g., 2, 3, 4, 5, 6, or more) ridges 122. The ridges 122 may be in the end cap proximal region 107 as shown in Figure 3C or they may exist in other regions, e.g., end cap distal region 109 or end cap reduced diameter region 114. The ridges 122 may taper between the maximum diameter 103 of the end cap 110 and the end cap reduced diameter region 114. The ridges 122 may have a perpendicular portion 126 and a tapered portion 124 to a center line of an elongate member (e.g., 102) as shown in Figure 3C. The tapered portion 124 may form an angle between 5 and 45 degrees from a centerline (e.g., of endcap 110 or elongate member 102). For example, the tapered portion 124 may be approximately 5, 10, 15, 20, 25, 30, 35, 40, or 45 degrees depending on application. The tapered portion 124 may have a larger effective diameter towards the distal end 118 than the proximal end 120 of endcap 110 or alternatively the tapered portion 124 may have a larger effective diameter towards the proximal end 120 than the distal end 118. In addition, the ridges 122 may include rounded or flattened edges. The ridges 122 may have a flattened edge with a range of approximately 0.02 mm to 0.2 mm or more. For example, a ridge 122 may have a flattened edge of 0.02mm, 0.06mm, 0.12mm, 0.14mm, 0.16mm, 0.18mm or 0.2mm. A ridge 122 may also have a rounded edge with a dimension ranging between approximately 0.02mm and 0.4mm. For example, a ridge 122 may have a rounded edge of approximately 0.02mm, 0.05mm, 0.08mm, 0.11 mm, 0.14mm, 0.17mm, 0.20mm, 0.23mm, 0.26mm, 0.29mm, 0.32mm, 0.35mm, .038, .041mm. The proximal end 120 of end cap 110 may abut the endoprosthesis 104. In certain instances, the endoprosthesis 104 may extend onto the proximal end 120 of endcap 110, and in other instances, the endoprosthesis 104 may extend up to the proximal end 120, as is shown in FIG. 3C. In certain instances, the ridges may be used to help a covering member 106 to resist movement. For example, when the endoprosthesis delivery system 100 is inserted through an introducer sheath or during manufacturing.

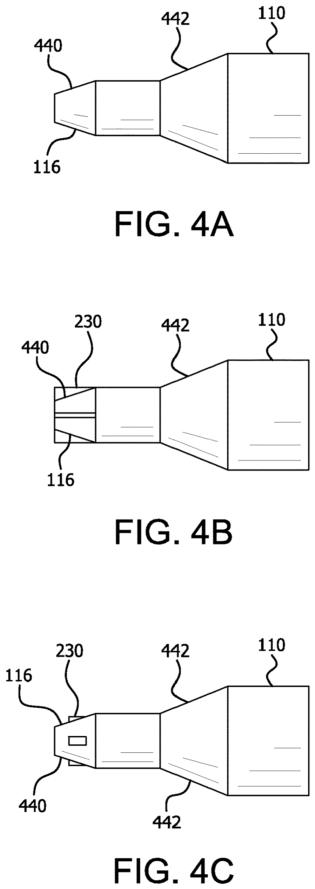

In certain instances, regions of the end cap can have various diameters. For example, as shown in Figures 3A-3C, the end cap proximal region 107 and the end cap distal region 109 have effective maximum diameters (105, 103) that are approximately equal. In Figures 4A-4C the end cap 110 has varying diameters. The ridges 122 may define the end cap proximal region maximum effective diameter 105. For example, as shown in Figure 3C, the ridges 122, define the end cap proximal region maximum effective diameter 105. The end cap proximal region maximum effective diameter 105 may be the same dimension as a constrained endoprosthesis 104 maximum effective diameter.

As shown in Figures 3A-3C, the covering member 106 may extend along the endoprosthesis 104 and end cap proximal region 107. The covering member 106 may lie essentially parallel to elongate member 102. The covering member may partially lie between ridges 122. In other cases, the covering member 106 may taper from a larger diameter along the endoprosthesis 104 to a smaller diameter along end cap 110. The covering member 106 may be removed along end cap 110 and endoprosthesis 104, thereby exposing endoprosthesis 104 and thus allowing the endoprosthesis to be expanded. In some cases, the covering member 106 may more easily be removed along end cap 110 and along a constrained endoprosthesis 104 (e.g., an endoprosthesis with a scalloped distal end) if the maximum effective diameters of the end cap 110 and endoprosthesis 104 are approximately equal. The endoprosthesis 104 may have a graft portion and a stent portion. The endoprosthesis 104 may also include stent portions that extend beyond the graft portion. These stent portions may include various shapes, such as scalloped ends. In other cases, it may be beneficial to have constrained endoprosthesis 104 have an effective maximum diameter that is greater than the effective maximum diameter of the end cap 110.

Profile 116 can comprise, for example a continuous taper. In other embodiments, with initial reference to Figure 4A, profile 116 can comprise a "staged" taper. For example, profile 116 can comprise one or more tapered segments 440 and 442. Tapered segments 440 and 442 can be separated by one or more non-tapered segments. Profiles 116 comprising any configuration and number of tapered segments are within the scope of the present disclosure.

In accordance with the claims, the end cap 110 further comprises one more fins 230. In such embodiments, fins 230 are positioned at the proximal end 120. For example, one or more fins 230 are located along profile 116 of proximal end 120. In various embodiments, fins 230 can comprise a protrusion from profile 116. For example, as end cap 110 and catheter tip 108 are retracted into outer shaft 150, the proximal end of outer shaft 150 can exert pressure on fins 230, causing them to fold over. By at least partially folding over, fins 230 may reduce the difficulty in retracting catheter tip 108 and end cap 110 back into outer shaft 150.

In various embodiments, fins 230 may provide a resistance to radial forces against an outer surface of end cap 110. For example, fins 230 may be subjected to a radial compressive force by endoprosthesis 104 and/or covering member 106. Fins 230 may resist such compressive forces and maintain a relatively constant diameter. In various embodiments, characteristics of fins 230 such as thickness, material, and length, among others, may be selected to provide sufficient resistance to radial forces while maintaining the ability of the fins to fold over when retracted into outer shaft 150.

With initial reference to Figures 4B and 4C, fins 230 can be positioned on end cap 110 in various configurations. For example, as illustrated in Figure 4B, fins 230 can traverse the length of the taper of profile 116. In other embodiments, as illustrated in Figure 4C, fins 230 can be positioned along a portion of the length of the taper of profile 116 (instead of the entire length of the taper). Further, fins 230 can be positioned in any combination of arrangements, including one or more fins 230 located along one or more different portions of the length of the taper of profile 116. Any positioning of fins 230 along profile 116, including combinations of different positions, is within the scope of the present disclosure.

In various embodiments, fins 230 can have the same or nearly the same diameter as cylindrical body 112. Such fins 230 can provide a constant diameter, and therefore a consistent surface of support, for covering member 106. For example, as illustrated in Figure 4C, fins 230 may comprise a diameter less than the diameter of cylindrical body 112. Although described as having the same diameter as cylindrical body 112, fins 230 can comprise any suitable diameter, including multiple portions or regions having different diameters.

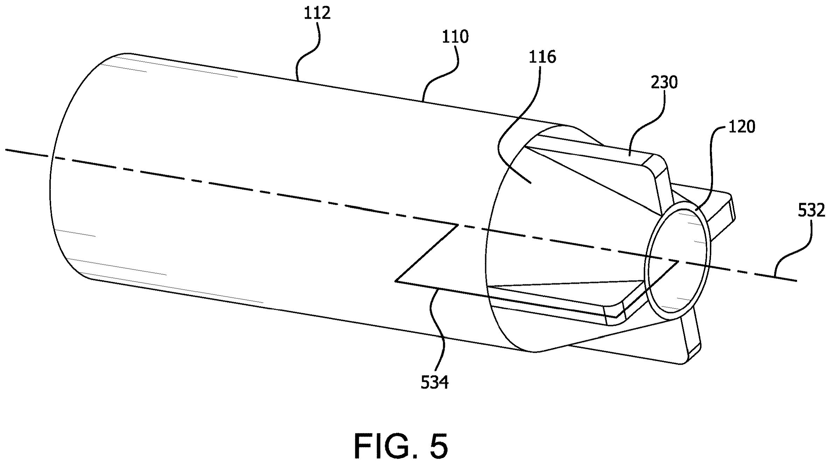

With initial reference to Figure 5, multiple fins 230 located at proximal end 120 of end cap 110 are illustrated. For example, to assist in understanding the spatial relationship and orientation of fins 230 and end cap 110, end cap 110 can comprise a longitudinal axis 532. In various embodiments, fins 230 are oriented such that a plane 534 parallel to fin 230 is parallel to and intersects with a portion of the length of longitudinal axis 532. Stated another way, in such embodiments, fins 230 are generally "straight" relative to longitudinal axis 532.

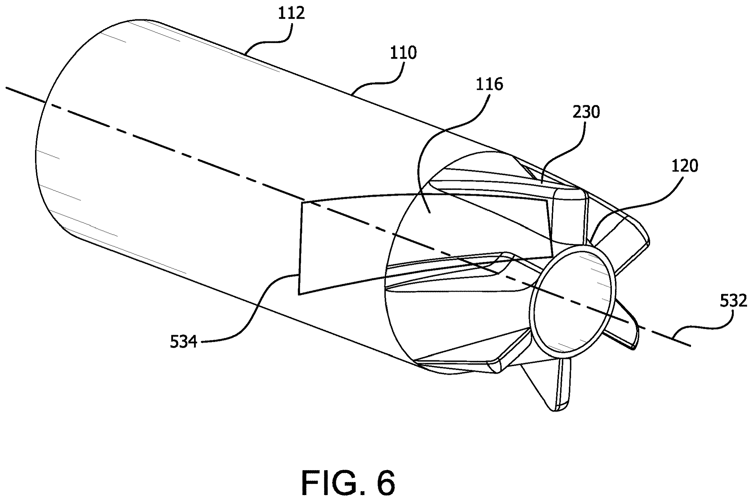

With initial reference to Figure 6, multiple fins 230 located at proximal end 120 of end cap 110 are illustrated. In various embodiments, fins 230 are oriented such that a plane 534 parallel to fin 230 is non-parallel to and intersecting at a point with longitudinal axis 532. Stated another way, in such embodiments, fins 230 are generally "angled" relative to longitudinal axis 532.

Although described in connection with two specific examples (e.g., the "straight" fins of Figure 5 and the "angled" fins of Figure 6), fins 230 can be configured in any way that provides support to covering member 106 at proximal end 120 of end cap 110. For example, fins 230 can be curved, bent, or otherwise non-straight. Any suitable configuration of fin 230 is in accordance with the present disclosure.

Numerous characteristics and advantages have been set forth in the preceding description, including various alternatives together with details of the structure and function of the devices and/or methods. The disclosure is intended as illustrative only and as such is not intended to be exhaustive. It will be evident to those skilled in the art that various modifications may be made, especially in matters of structure, materials, elements, components, shape, size, and arrangement of parts including combinations within the principles of the invention, to the full extent indicated by the broad, general meaning of the terms in which the appended claims are expressed. To the extent that these various modifications do not depart from the scope of the appended claims, they are intended to be encompassed therein.

Further, any combination of the elements and components of the present disclosure is within the scope of the present invention. Moreover, where a phrase similar to "at least one of A, B, or C" is used in the claims, it is intended that the phrase be interpreted to mean that A alone may be present in an embodiment, B alone may be present in an embodiment, C alone may be present in an embodiment, or that any combination of the elements A, B and C may be present in a single embodiment; for example, A and B, A and C, B and C, or A and B and C.

## Claims

1. A combined tip and end cap (108, 110) for an endoprosthesis delivery system (100):
the endoprosthesis delivery system comprising:
an elongate member (102),
an endoprosthesis (104) having a distal end, and
a covering member (106) surrounding a portion of the endoprosthesis and having a distal end that extends beyond the distal end of the endoprosthesis;
wherein the end cap (110) of the combined tip and end cap (108, 110) is configured to be coupled to the elongate member of the endoprosthesis delivery system adjacent to the distal end of the endoprosthesis; and
the end cap of the combined tip and end cap comprises a cylindrical body (112) and
a proximal end (120) having a tapered profile, and **characterised in that** one or more fins (230) are (290). located along the tapered profile (116).

2. The combined tip and end cap (108, 110) of claim 1, wherein the one or more fins (230) comprise protrusions from the tapered profile (116).

3. The combined tip and end cap (108, 110) of claim 2, wherein a length of the proximal end (120) having the tapered profile (116) exceeds the length of the one or more fins (230).

4. The combined tip and end cap (108, 110) of claim 2 or 3, wherein the tapered profile (116) comprises a plurality of fins (230) having a plurality of portions or regions with different diameters.

5. The combined tip and end cap (108, 110) of any one of claims 1-4, wherein the tapered profile (116) includes a first tapered segment (440) and a second tapered segment (442), wherein the first tapered segment and the second tapered segment are separated by one or more non-tapered segments.

6. The combined tip and end cap (108, 110) of claim 5, wherein the first tapered segment (440) has a diameter no greater than a diameter of the second tapered segment (442).

7. The combined tip and end cap (108, 110) of claim 6, wherein the one or more fins (230) are disposed at the first tapered segment (440) which is located proximally with respect to the second tapered segment (442).

8. The combined tip and end cap (108, 110) of any one of claims 5-7, wherein each of the non-tapered segments has a constant diameter.

9. The combined tip and end cap (108, 110) of any one of claims 1-8, wherein the tapered profile (116) is proximally tapering at an angle from approximately 5 to 45 degrees with respect to a centerline of the end cap of the combined tip and end cap.

10. An endoprosthesis delivery system (100) comprising:
an elongate member (102),
an endoprosthesis (104) having a distal end,
a combined tip and end cap (108, 110) according to any preceding claim coupled to the elongate member adjacent the distal end of the endoprosthesis; and
a covering member (106) surrounding a portion of the endoprosthesis and having a distal end that extends beyond the distal end of the endoprosthesis.

## Patentansprüche

1. Kombinierte Spitze und Endkappe (108, 110) für ein Endoprotheseneinführungssystem (100):
wobei das Endoprotheseneinführungssystem Folgendes umfasst:
ein längliches Element (102),
eine Endoprothese (104), die ein distales Ende aufweist, und
ein Abdeckelement (106), das einen Abschnitt der Endoprothese umgibt und ein distales Ende aufweist, das sich über das distale Ende der Endoprothese hinaus erstreckt;
wobei die Endkappe (110) der kombinierten Spitze und Endkappe (108, 110) dazu konfiguriert ist, an das längliche Element des Endoprotheseneinführungssystems angrenzend an das distale Ende der Endoprothese gekoppelt zu werden; und
die Endkappe der kombinierten Spitze und Endkappe einen zylindrischen Körper (112) und ein proximales Ende (120), das ein sich verjüngendes Profil aufweist, umfasst, und **dadurch gekennzeichnet, dass** sich eine oder mehrere Lamellen (230) entlang des sich verjüngenden Profils (116) befinden.

2. Kombinierte Spitze und Endkappe (108, 110) nach Anspruch 1, wobei die eine oder mehreren Lamellen (230) Vorsprünge von dem sich verjüngenden Profil (116) umfassen.

3. Kombinierte Spitze und Endkappe (108, 110) nach Anspruch 2, wobei eine Länge des proximalen Endes (120), das das sich verjüngende Profil (116) aufweist, die Länge der einen oder mehreren Lamellen (230) übersteigt.

4. Kombinierte Spitze und Endkappe (108, 110) nach Anspruch 2 oder 3, wobei das sich verjüngende Profil (116) eine Vielzahl von Lamellen (230) mit einer Vielzahl von Abschnitten oder Bereichen mit unterschiedlichen Durchmessern umfasst.

5. Kombinierte Spitze und Endkappe (108, 110) nach einem der Ansprüche 1 bis 4, wobei das sich verjüngende Profil (116) ein erstes sich verjüngendes Segment (440) und ein zweites sich verjüngendes Segment (442) beinhaltet, wobei das erste sich verjüngende Segment und das zweite sich verjüngende Segment durch ein oder mehrere sich nicht verjüngende Segmente getrennt sind.

6. Kombinierte Spitze und Endkappe (108, 110) nach Anspruch 5, wobei das erste sich verjüngende Segment (440) einen Durchmesser aufweist, der nicht größer als ein Durchmesser des zweiten sich verjüngenden Segments (442) ist.

7. Kombinierte Spitze und Endkappe (108, 110) nach Anspruch 6, wobei die eine oder mehreren Lamellen (230) an dem ersten sich verjüngenden Segment (440) angeordnet sind, das sich proximal in Bezug auf das zweite sich verjüngende Segment (442) befindet.

8. Kombinierte Spitze und Endkappe (108, 110) nach einem der Ansprüche 5 bis 7, wobei jedes der sich nicht verjüngenden Segmente einen konstanten Durchmesser aufweist.

9. Kombinierte Spitze und Endkappe (108, 110) nach einem der Ansprüche 1 bis 8, wobei sich das sich verjüngende Profil (116) in einem Winkel von etwa 5 bis 45 Grad in Bezug auf eine Mittellinie der Endkappe der kombinierten Spitze und Endkappe proximal verjüngt.

10. Endoprotheseneinführungssystem (100), umfassend:
ein längliches Element (102),
eine Endoprothese (104), die ein distales Ende aufweist,
eine kombinierte Spitze und Endkappe (108, 110) nach einem vorhergehenden Anspruch, die an das längliche Element angrenzend an das distale Ende der Endoprothese gekoppelt ist; und
ein Abdeckelement (106), das einen Abschnitt der Endoprothese umgibt und ein distales Ende aufweist, das sich über das distale Ende der Endoprothese hinaus erstreckt.

## Revendications

1. Pointe et capuchon d'extrémité combinés (108, 110) destinés à un système de placement d'endoprothèse (100) :
le système de placement d'endoprothèse comprenant :
un élément allongé (102),
une endoprothèse (104) possédant une extrémité distale, et
un élément de recouvrement (106) entourant une partie de l'endoprothèse et possédant une extrémité distale qui s'étend au-delà de l'extrémité distale de l'endoprothèse ;
ledit capuchon d'extrémité (110) de la pointe et du capuchon d'extrémité combinés (108, 110) étant conçu pour être couplé à l'élément allongé du système de placement d'endoprothèse adjacent à l'extrémité distale de l'endoprothèse ; et
ledit capuchon d'extrémité de la pointe et du capuchon d'extrémité combinés comprenant un corps cylindrique (112) et une extrémité proximale (120) présentant un profil effilé, et **caractérisé en ce qu'**une ou plusieurs ailettes (230) sont situées le long du profil effilé (116).

2. Pointe et capuchon d'extrémité combinés (108, 110) selon la revendication 1, lesdites une ou plusieurs ailettes (230) comprenant des saillies à partir du profil effilé (116).

3. Pointe et capuchon d'extrémité combinés (108, 110) selon la revendication 2, la longueur de l'extrémité proximale (120) présentant le profil effilé (116) dépassant la longueur desdites une ou plusieurs ailettes (230).

4. Pointe et capuchon d'extrémité combinés (108, 110) selon la revendication 2 ou 3, ledit profil effilé (116) comprenant une pluralité d'ailettes (230) comportant une pluralité de parties ou de zones de diamètres différents.

5. Pointe et capuchon d'extrémité combinés (108, 110) selon l'une quelconque des revendications 1 à 4, ledit profil effilé (116) comprenant un premier segment effilé (440) et un second segment effilé (442), ledit premier segment effilé et ledit second segment effilé étant séparés par un ou plusieurs segments non effilés.

6. Pointe et capuchon d'extrémité combinés (108, 110) selon la revendication 5, ledit premier segment effilé (440) présentant un diamètre qui n'est pas supérieur au diamètre du second segment effilé (442).

7. Pointe et capuchon d'extrémité combinés (108, 110) selon la revendication 6, lesdites une ou plusieurs ailettes (230) étant disposées au niveau du premier segment effilé (440) qui est situé de manière proximale par rapport au second segment effilé (442).

8. Pointe et capuchon d'extrémité combinés (108, 110) selon l'une quelconque des revendications 5 à 7, chacun des segments non effilés présentant un diamètre constant.

9. Pointe et capuchon d'extrémité combinés (108, 110) selon l'une quelconque des revendications 1 à 8, ledit profil effilé (116) se rétrécissant de manière proximale selon un angle d'approximativement 5 à 45 degrés par rapport à une ligne centrale du capuchon d'extrémité de la pointe et du capuchon d'extrémité combinés.

10. Système de placement d'endoprothèse (100) comprenant :
un élément allongé (102),
une endoprothèse (104) possédant une extrémité distale,
une pointe et un capuchon d'extrémité combinés (108, 110) selon l'une quelconque des revendications précédentes couplés à l'élément allongé adjacent à l'extrémité distale de l'endoprothèse ; et
un élément de recouvrement (106) entourant une partie de l'endoprothèse et possédant une extrémité distale qui s'étend au-delà de l'extrémité distale de l'endoprothèse.
